# EUROPÄISCHE PATENTANMELDUNG

(11) **EP 1 579 803 A1**
(43) Veröffentlichungstag der Anmeldung: **28.09.2005**
(21) Anmeldenummer: 04007094.8
(22) Anmeldetag: 24.03.2004
(51) Int. Cl.: A61B 5/103, A61B 5/107

(54) **Verfahren und Vorrichtung zum Ermitteln einer Position eines charakteristischen Punktes**

(71) Anmelder: BrainLAB AG, 85551 Kirchheim/Heimstetten (DE)
(72) Erfinder: Dick, Robert, 81827 München (DE)
(74) Vertreter: Schwabe - Sandmair - Marx

(57) **Zusammenfassung**

Die Erfindung bezieht sich auf ein Verfahren zum Ermitteln einer Position eines charakteristischen Punktes einer Körperstruktur 5, wobei die Positionen einer Mehrzahl von Punkten im Bereich des charakteristischen Punktes erfasst werden und aus den erfassten Positionen der Mehrzahl der Punkte die Position des charakteristischen Punktes ermittelt wird, sowie auf eine Vorrichtung zum Ermitteln einer Position eines charakteristischen Punktes einer Körperstruktur mit einer Kamera 2, welche die Position eines Pointers 1 erfassen kann und welche mit einer Recheneinheit 3 gekoppelt ist, wobei die Recheneinheit 3 aus einer Mehrzahl von Punkten, welche von der Kamera 2 erfasst wurden, die Position eines charakteristischen Punktes einer Körperstruktur 5 berechnet.

## Beschreibung

Die Erfindung bezieht sich auf ein Verfahren und eine Vorrichtung zum Ermitteln einer Position eines charakteristischen Punktes einer Körperstruktur, wie z. B. einer Hüfte, wobei solche charakteristischen Punkte auch als anatomische Landmarks bezeichnet werden und zum Registrieren einer z. B. durch eine Computertomographie aufgenommenen Körperstruktur verwendet werden.

Zum Akquirieren eines charakteristischen Punktes bzw. einer Landmark einer Körperstruktur wird nach einem bekannten Verfahren die Spitze eines Pointers, an welchem Marker zur Positionserfassung angebracht sind, zu einer Stelle bewegt, welche vorher durch manuelles Abtasten (Palpating) ermittelt wurde. Sobald die Spitze des Pointers auf der durch Abtasten aufgefundenen charakteristischen Körperstruktur liegt, wird der Pointer geschwenkt, um so diesen charakteristischen Punkt aufzunehmen. Dabei spielt die Fähigkeit eines Chirurgen den gewünschten Punkt durch Abtasten aufzufinden und den Pointer exakt zu positionieren eine entscheidende Rolle, so dass dieses Verfahren zum Auffinden einer anatomischen Landmark oft ungenau und nicht reproduzierbar ist, da sich häufig Abweichungen bei den so aufgenommenen Positionen der Landmarks ergeben.

Es ist eine Aufgabe der Erfindung, ein Verfahren und eine Vorrichtung vorzuschlagen, mit welchen die Akquisition von charakteristischen Körperstrukturpunkten verbessert werden kann.

Diese Aufgabe wird durch das Verfahren und die Vorrichtung wie in den unabhängigen Ansprüchen definiert gelöst. Vorteilhafte Ausführungsformen ergeben sich aus den Unteransprüchen.

Nach dem erfindungsgemäßen Verfahren zum reproduzierbaren Ermitteln einer Position eines oder mehrerer charakteristischen Punkte einer Körperstruktur, also z. B. der Position von Landmarks einer im Körper liegenden Struktur, wie z. B. einer Hüfte, wird zunächst die Position einer Mehrzahl von Punkten im Bereich des charakteristischen Punktes bzw. der Landmark erfasst. Hierzu kann z. B. auf manuelle Art durch Abtasten (Palpating) an der Körperoberfläche oder Haut der Bereich ermittelt werden, in welchem sich der charakteristische Punkt bzw. die Landmark befindet. Nachdem der Bereich ermittelt wurde, kann die z. B. stumpfe Spitze eines kalibrierten Pointers zu einer Vielzahl von Stellen innerhalb des Bereichs auf der Haut oder Körperoberfläche bewegt werden, um so die Position einer Mehrzahl von Punkten auf der Körperoberfläche bzw. Haut im Bereich der Landmark zu erfassen. Erfindungsgemäß wird aus der so erfassten Position der verschiedenen aufgenommenen Punkte, welche eine Punktwolke z. B. in Form eines Teils einer Kugeloberfläche bilden, der charakteristische Punkt oder die Landmark ermittelt. Beispielsweise kann als charakteristischer Punkt der prominenteste oder am weitesten vorstehende Punkt als Landmark ermittelt werden. Insbesondere ist es z. B. einfach eine Landmark aus einer solchen Vielzahl von aufgenommenen Punkten oder aus einer Punktwolke zu bestimmen, wenn diese Landmark das Ende einer Kante der Körperstruktur ist.

Somit ist es durch das erfindungsgemäße Verfahren möglich, dass ein charakteristischer Punkt bzw. eine Landmark auf Grund der Verwendung mehrerer akquirierter Punkte mit größerer Genauigkeit ermittelt werden kann, wobei eine geringere Wahrscheinlichkeit einer ungenauen Punktakquisition besteht, so dass das erfindungsgemäße Verfahren unabhängig von den Fertigkeiten eines Chirurgen mit sehr guter Reproduzierbarkeit durchgeführt werden kann. Eine anatomische Landmark kann also durch die Aufnahme mehrerer Punktpositionen bestimmt und digitalisiert werden.

Vorteilhaft wird die Mehrzahl der Punkte dadurch erfasst, dass auf der Körperoberfläche, also z. B. der Haut oder auf einem auf der Haut aufliegenden Stoff, die Spitze eines bevorzugt stumpfen Pointers zu verschiedenen Punkten innerhalb eines Bereiches bewegt wird, wobei die Haut nicht verletzt oder durchstochen wird, so dass das Verfahren zum Aufnehmen von Punkten für einen Patienten schmerzfrei durchgeführt werden kann. Jedoch ist es prinzipiell auch möglich, dass die Spitze eines Pointers durch die Haut hindurch direkt auf die Körperstruktur, wie z. B. die Hüfte, bewegt wird, um eine Mehrzahl von Punkten auf der Körperstruktur selbst aufzunehmen, so dass daraus die Position einer oder mehrerer Landmarks ermittelt werden kann.

Werden die zur Durchführung des erfindungsgemäßen Verfahrens aufgenommenen Punkte an der Hautoberfläche aufgenommen, so kann zur Ermittlung der Position der Landmark bzw. des charakteristischen Punktes die Dicke der Haut bei der Berechnung berücksichtigt werden, so dass beispielsweise als Landmark der Körperstruktur ein Punkt errechnet wird, welcher im Abstand der Dicke der Haut unter dem Punkt liegt, welcher auf der Hautoberfläche als charakteristischer und z. B. am weitesten vorstehender Punkt ermittelt wurde. Somit kann z. B. bei Verwendung von bekannten Referenzmodellen der Körperstruktur ohne eine CT-Aufnahme die Körperstruktur registriert werden.

Vorteilhaft werden mehrere charakteristische Punkte oder Landmarks einer Körperstruktur durch das oben beschriebene Verfahren ermittelt, wobei es besonders vorteilhaft ist, solche Punkte als Landmarks zu verwenden, welche auf einer symmetrischen Körperstruktur oder auf einer charakteristischen Ebene oder Achse liegen wie z. B. zwei Punkte auf einer Hüfte, welche z.B. symmetrisch zur Symmetrieebene oder Mittsaggitalebene der Hüfte liegen. Damit kann beispielsweise aus der Position von zwei solchen ermittelten Landmarks die z. B. bei der Planung einer Hüftoperation zu bestimmende Mittsaggitalebene als Symmetrieebene zu diesen Punkten berechnet werden.

Bevorzugt wird das erfindungsgemäße Verfahren verwendet, um Landmarks im Bereich der Hüfte, der Hand, des Armes, der Beine, insbesondere der Knie, oder der Wirbelsäule zu ermitteln. Allgemein ist das erfindungsgemäße Verfahren jedoch geeignet, um bei jeder beliebigen Körperstruktur charakteristische Punkte oder Landmarks mit guter Reproduzierbarkeit zu bestimmen.

Vorteilhaft können die nach dem obigen Verfahren ermittelten Positionen von einem oder mehreren Punkte bei einem sogenannten Paired Point Match verwendet werden, wobei eine virtuelle Körper- oder Knochenstruktur, welche z. B. durch eine Computertomographieaufnahme erhalten wurde, mit der realen Körper- oder Knochenstruktur abgeglichen wird, um so die Körperstruktur zu registrieren. Dabei wird ein charakteristischer Punkt aus der virtuellen Körperstruktur einem charakteristischen Punkt der realen Körperstruktur, dessen Position nach dem oben beschriebenen Verfahren ermittelt wurde, zugeordnet, so dass nach der paarweisen Zuordnung von einem oder mehreren Punkten die Lage der realen Körper- oder Knochenstruktur im Raum bekannt ist, diese also registriert ist.

Ebenso können die Positionen eines oder mehrerer charakteristischer Punkte, welche nach dem obigen Verfahren ermittelt wurden, oder auch einer oder mehrerer aufgenommener und einer Körperstruktur zugeordneter Punktwolken verwendet werden, um einen Abgleich bzw. eine Registrierung einer Körper- oder Knochenstruktur durchzuführen, wobei die Punkte oder Punktwolken, welche einen Oberflächen- oder Hautbereich des Körpers beschreiben, z. B. um die Dicke der Haut geschrumpft werden können, um die Oberfläche der Körperstruktur selbst abzubilden.

Des weiteren können vorteilhaft in einem Fall wenn z.B. keine CT-Daten vorliegen die nach dem obigen Verfahren ermittelten Positionen eines oder mehrere Punkte oder auch Punktwolken verwendet werden, um z. B. bei einer Körper- oder Knochenstruktur, bei welcher schon nach dem obigen Verfahren die Position eines Punktes oder einer Punktwolke ermittelt wurde, eine Registrierung dadurch durchzuführen, dass die bereits aufgenommene Position eines charakteristischen Punktes oder einer Punktwolke mit der zur Registrierung der Körper- oder Knochenstruktur aufgenommenen oder ermittelten Position eines oder mehrerer charakteristischen Punkte oder Punktwolken abgeglichen wird.

Weiterhin bezieht sich die Erfindung auf ein Computerprogramm, welches, wenn es in einen Computer geladen ist oder auf einem Computer läuft, ein oder mehrere der oben beschriebenen Verfahrensschritte durchführt. Ebenso bezieht sich die Erfindung auf ein Programmspeichermedium oder ein Computerprogrammprodukt mit einem solchen Programm.

Gemäß einem weiteren Aspekt bezieht sich die Erfindung auf eine Vorrichtung zum Ermitteln einer Position mindestens eines charakteristischen Punktes einer Körperstruktur mit einem Navigationssystem bestehend aus einer Recheneinheit bzw. einem Computer, an welchem eine Kamera angeschlossen ist, mit welcher die Position eines Pointers erfasst werden kann. In dem Navigationssystem kann die Geometrie des Pointers eingegeben werden oder bereits gespeichert sein, so dass der Pointer präkalibriert ist und die Position der Spitze des Pointers berechnet werden kann. Die Recheneinheit bzw. der Computer des Navigationssystems kann aus einer Mehrzahl von im Bereich eines charakteristischen Punktes erfassten Positionen einzelner Punkte die Position des charakteristischen Punktes nach dem oben beschriebenen Verfahren ermitteln und kann vorteilhaft diese Position und/oder die Positionen von Punktwolken abspeichem, um diese z. B. bei einem nachfolgenden Registrierungsverfahren zu verwenden.

Vorteilhaft können in die Vorrichtung Daten eingegeben werden, welche aus einer vorherigen Aufnahme der Körperstruktur z. B. mittels eines Computertomographen stammen, um z. B. das oben beschriebene Paired Point Verfahren durchführen zu können.

Die Erfindung wird nachfolgend anhand eines Ausführungsbeispiels beschrieben. Es zeigt:

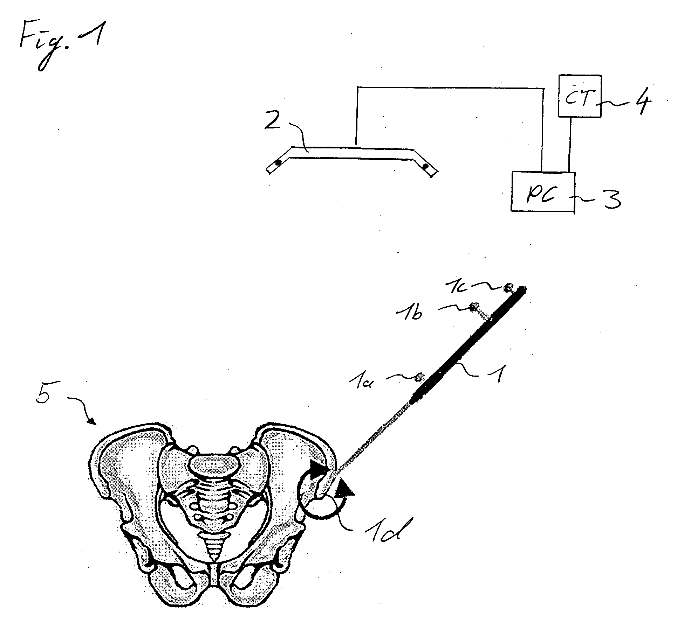

- Figur 1: eine erfindungsgemäße Vorrichtung, mit welcher eine Position eines charakteristischen Punktes einer Hüfte ermittelt werden kann.

Figur 1 zeigt schematisch eine erfindungsgemäße Vorrichtung mit einem Navigationssystem, das eine Infrarotkamera 2 und einen Computer 3 aufweist, wobei die Infrarotkamera 2 mit dem Computer 3 gekoppelt ist. In den Computer 3 können Daten eingegeben werden, welche von einer Aufnahme der Hüfte in einem Computertomographen 4 stammen.

Mit der Infrarotkamera 2 kann auf bekannte Art die Position eines Pointers 1 erfasst werden, der drei als reflektierende Marker dienende Kugeln 1a, 1b und 1c aufweist, die fest mit dem Pointer 1 verbunden sind. Ist der Pointer 1 kalibriert, so kann aus der erfassten Position der drei Marker 1a, 1b und 1c die Position der Spitze 1 d des Pointers ermittelt werden.

Mit der Spitze 1b des Pointers 1 können z. B. mehrere Punkte aufgenommen werden, welche der schematisch dargestellten Hüfte 5 zugeordnet sind, indem z. B. die Spitze 1d des Pointers 1 zu einem Bereich der Haut bewegt wird, welcher über dem aufzufindendem charakteristischen Punkt der Hüfte 5 liegt. Durch ein Schwenken des Pointers 1 an einer festen Stelle der Pointerspitze 1d kann das Erfassungs- oder Akquisitionsverfahren gestartet werden. In diesem Bereich auf der Haut wird die Pointerspitze 1 d z. B. durch den Finger eines Arztes auf die Haut gedrückt und durch eine z. B. kreisende Bewegung des Fingers über den Bereich der Haut bewegt, unter welchem sich der charakteristische Punkt befindet. So können eine Vielzahl von Punkten als Punktwolke von der Infrarotkamera 2 aufgenommen und im Computer 3 abgespeichert werden, so dass der Computer 3 aus der so aufgenommenen Punktwolke die Position eines charakteristischen Punktes der Hüfte 5, wie z. B. eine Endposition einer Kante der Hüfte 5, ermitteln kann.

## Patentansprüche

1. Verfahren zum Ermitteln einer Position eines charakteristischen Punktes einer Körperstruktur (5), wobei die Positionen einer Mehrzahl von Punkten im Bereich des charakteristischen Punktes erfasst werden und aus den erfassten Positionen der Mehrzahl der Punkte die Position des charakteristischen Punktes ermittelt wird.

2. Verfahren nach Anspruch 1, wobei die Positionen der Mehrzahl der Punkte auf der Haut eines Patienten erfasst wird.

3. Verfahren nach einem der vorhergehenden Ansprüche, wobei zur Ermittlung der Position des charakteristischen Punktes der Körperstruktur die Hautdicke berücksichtigt wird.

4. Verfahren nach einem der vorhergehenden Ansprüche, wobei die Positionen von mehreren charakteristischen Punkten ermittelt werden.

5. Verfahren nach einem der vorhergehenden Ansprüche, wobei die Positionen charakteristischer Punkte einer Hüfte, einer Hand, eines Arms, eines Beins, eines Knies oder einer Wirbelsäule ermittelt werden.

6. Verfahren nach einem der vorhergehenden Ansprüche, wobei die ermittelte Position mindestens eines charakteristischen Punktes und/oder die Positionen der Mehrzahl der Punkte zum Abgleich mit einem vorher aufgenommenen Modell der Körperstruktur verwendet wird.

7. Verfahren nach einem der vorhergehenden Ansprüche, wobei die Position mindestens eines charakteristischen Punktes und/oder die Positionen der Mehrzahl der Punkte zum Registrieren einer Körperstruktur verwendet werden.

8. Computerprogramm, welches, wenn es in einen Computer geladen ist oder auf einem Computer läuft, das Verfahren nach einem der vorhergehenden Ansprüche durchführt.

9. Programmspeichermedium oder Computerprogrammprodukt mit dem Programm nach dem vorhergehenden Anspruch.

10. Vorrichtung zum Ermitteln einer Position eines charakteristischen Punktes einer Körperstruktur mit einer Kamera (2), welche die Position eines Pointers (1) erfassen kann und welche mit einer Recheneinheit (3) gekoppelt ist, wobei die Recheneinheit (3) aus einer Mehrzahl von Punkten, welche von der Kamera (2) erfasst wurden, die Position eines charakteristischen Punktes einer Körperstruktur (5) berechnen kann.
